# EUROPEAN PATENT APPLICATION

(11) **EP 2 762 560 A1**
(43) Date of publication of application: **06.08.2014**
(21) Application number: 12837508.6
(22) Date of filing: 28.09.2012
(51) Int. Cl.: C12N 5/0735, C12N 5/0775, C12N 5/0793, C12N 5/0797, C12N 5/10, C12Q 1/02, G01N 33/15, G01N 33/50

(54) **METHOD FOR INDUCING OREXIN NEURON**

(30) Priority: 29.09.2011 US 201161540601 P
(71) Applicant: The University of Tokyo, Bunkyo-ku, Tokyo 113-8654 (JP)
(72) Inventor: SHIOTA, Kunio, Tokyo 113-8654 (JP); YAGI, Shintaro, Tokyo 113-8654 (JP); HAYAKAWA, Koji, Tokyo 113-8654 (JP); HIROSAWA-TAKAMORI, Mitsuko, Tokyo 113-8654 (JP); ARAI, Daisuke, Tokyo 113-8654 (JP); HIRABAYASHI, Keiji, Tokyo 113-8654 (JP)
(74) Representative: Woods, Geoffrey Corlett
(86) International application number: PCT/JP2012/075137
(87) International publication number: WO 2013/047773

(57) **Abstract**

Provided is a means for inducing an orexin neuron. Provided is a method for producing an orexin neuron containing a step of culturing a pluripotent stem cell or a neural progenitor cell in the presence of N-acetyl-D-mannosamine. Also provided is a method for producing an orexin neuron containing a step of culturing a pluripotent stem cell or a neural progenitor cell in the presence of N-acetyl-D-mannosamine, and at least one inhibitor selected from the group consisting of a Sirtuin inhibitor and an O-linked β-N-acetylglucosamine transferase inhibitor.

## Description

### TECHNICAL FIELD

The present invention relates to a method for inducing an orexin neuron, more particularly, a method for efficiently producing an orexin neuron from a pluripotent stem cell or a neural progenitor cell.

### BACKGROUND ART

Orexin is an intracerebral peptide which promotes wakefulness, and constitutes an orexin regulatory system and plays the central function of regulating wakefulness-sleep via an orexin receptor which is widely distributed in the brain (Non-Patent Document 1). Experimentally, lack of orexin leads to narcolepsy (a pathological condition in which a person suddenly and paroxysmally falls into short sleep) (Non-Patent Documents 2 and 3). Further, the concentration of orexin in the cerebrospinal fluid of a narcolepsy patient is as low as about 1/3 of that of a healthy person, and decrease in the number of orexin neurons is also observed. It has been revealed that the hypofunction of the orexin system influences on the wakefulness-sleep cycle, and also adversely influences on maintenance of homeostasis. The proper activity of the orexin system is essential for maintaining normal wakefulness-sleep and maintaining homeostasis (Non-Patent Document 4). For this reason, development of an orexin action promoter (agonist) or inhibitor (antagonist) has been attracting attention. On the other hand, when an orexin producing cell (orexin neuron) is absent or decreased, effectiveness of such agonists or antagonits is limited. However, until now, there is no report on successful methods to induce an orexin neuron in vivo or in vitro.

N-acetyl-D-mannosamine, an isomer of N-acetyl-D-glucosamine, is known as, for example, a starting material for the enzymatic synthesis of sialic acid (N-acetyl-neuraminic acid), which serves as a medicament and a starting material for other medicaments. Also, N-acetyl-D-mannosamine permits enzymatic synthesis of sialic acid derivatives from derivatives thereof, hence an industrially important substance. In a known method of producing N-acetyl-D-mannosamine, the molar conversion yield of N-acetyl-mannosamine from N-acetylglucosamine in isomerizing the latter under alkaline conditions is increased by the addition of boric acid or borate (patent reference 1). Another known method is such that sialic acid, as the substrate, is reacted with N-acetyl-neuraminate lyase to produce N-acetyl-D-mannosamine (patent reference 2). A method has been proposed wherein the acylated form of N-mannosamine is contacted with cells to regulate lectin binding to cell surfaces or to regulate the proliferation of nerve cells (patent reference 3).

The present inventors have found that N-acetyl-D-mannosamine is effective in ameliorating brain hypofunction and in ameliorating sleep disorders (patent documents 4 and 5).

### [Document List]

### [patent documents]

patent document 1: JP-A-HEI-10-182685
patent document 2: JP-A-2001-78794
patent document 3: US Patent No. 6274568
patent document 4: WO2010/027028
patent document 5: JP-A-2011-178702

### [non-patent documents]

non-patent document 1: Pharmacological Reviews 61:162-176, 2009
non-patent document 2: Cell 98: 365-376, 1999
non-patent document 3: Cell 98: 437-451, 1999
non-patent document 4: Neuroscience 178: 82-88, 2011

### SUMMARY OF THE INVENTION

### Problems to be Solved by the Invention

Destabilization of wakefulness-sleep causes a serious brain dysfunction such as reduction in cognitive or learning capacity, and leads to deterioration in the brain function. Previously, induction of sleep has been tried by a sleep-inducing drug aiming at improvement in sleep. The orexin action promoter or inhibitor is an extension thereof. However, particularly, the problem of wakefulness-sleep accompanied with aging is a problem of reduction in the quality of sleep or destabilization of wakefulness-sleep rather than decrease in the sleeping time. The orexin system is essential for stabilization of wakefulness-sleep. When it becomes possible to induce an orexin neuron, this provides a new treatment method covering diseases exhibiting abnormality of the wakefulness-sleep cycle. An object of the present invention is to provide a means for inducing an orexin neuron in vitro.

### SOLUTIONS TO THE PROBLEMS

The present inventors extensively investigated to solve the above-described problems and, as a result, found out that by adding N-acetyl-D-mannosamine, or a combination of N-acetyl-D-mannosamine with a particular inhibitor to a medium during the process of differentiation into a nerve cell, an orexin neuron can be induced from a pluripotent stem cell or a neural progenitor cell, resulting in completion of the present invention. That is, the present invention is as follows.
[1] A method for producing an orexin neuron, comprising a step of culturing a pluripotent stem cell or a neural progenitor cell in the presence of N-acetyl-D-mannosamine.
[2] The method according to [1], wherein the pluripotent stem cell is an embryonic stem cell, a somatic stem cell or an induced pluripotent stem cell.
[3] The method according to [1], wherein the neural progenitor cell is a fetus-derived neurosphere.
[4] An orexin neuron, which is obtainable by the method according to any of [1] to [3].
[5] A method for screening for a drug which acts on regulation of wakefulness-sleep, comprising using the orexin neuron according to [4].
[6] The screening method according to [5], wherein the drug is a therapeutic agent for narcolepsy.
[7] A method for inducing an orexin neuron, comprising a step of administering an effective amount of N-acetyl-D-mannosamine to a subject in need thereof, or having an effective amount of N-acetyl-D-mannosamine taken by a subject in need thereof.
[8] An agent for inducing an orexin neuron from a neural progenitor cell or a nerve cell, comprising N-acetyl-D-mannosamine as an active ingredient.
[9] A method for producing an orexin neuron, comprising a step of culturing a pluripotent stem cell or a neural progenitor cell in the presence of N-acetyl-D-mannosamine, and at least one inhibitor selected from the group consisting of a Sirtuin (Sirt) inhibitor and an O-linked β-N-acetylglucosamine transferase (OGT) inhibitor.
[10] The method according to [9], wherein the pluripotent stem cell is an embryonic stem cell, a somatic stem cell or an induced pluripotent stem cell.
[11] The method according to [9], wherein the neural progenitor cell is a fetus-derived neurosphere.
[12] An orexin neuron, which is obtainable by the method according to any of [9] to [11].
[13] A method for screening for a drug which acts on regulation of wakefulness-sleep, comprising using the orexin neuron according to [12].
[14] The screening method according to [13], wherein the drug is a therapeutic agent for narcolepsy.
[15] An agent for inducing an orexin neuron from a neural progenitor cell or a nerve cell, comprising N-acetyl-D-mannosamine, and at least one inhibitor selected from the group consisting of a Sirtuin 1 (Sirt1) inhibitor and an O-linked β-N-acetylglucosamine transferase (OGT) inhibitor as active ingredients.

### EFFECTS OF THE INVENTION

The present inventors first demonstrated that addition of ManNAc (including a derivative, a precursor or a prodrug thereof) or a combination of ManNAc and a Sirt inhibitor and/or an Ogt (O-linked β-N-acetylglucosamine (O-GlcNAc) transferase) inhibitor has the action of inducing an orexin neuron in a culture system of a pluripotent stem cell including an ES cell, or a neural progenitor cell.

ManNAc (including a derivative, a precursor or a prodrug thereof) or a combination of ManNAc and a Sirt inhibitor and/or an Ogt inhibitor is effective for diseases exhibiting symptoms due to decrease or lack of an orexin neuron. An orexin neuron produced using ManNAc (including a derivative, a precursor or a prodrug thereof) or a combination of ManNAc and a Sirt inhibitor and/or an Ogt inhibitor can provide a new drug therapy or drug screening. The present invention can also contribute to regeneration medicine aiming at recovery of an orexin neuron.

### BRIEF DESCRIPTION OF THE DRAWINGS

Fig. 1 shows a schedule of induction of neural differentiation from a mouse ES cell using a SDIA differentiation culture system.
Fig. 2 shows expression of a Hcrt gene in neurally differentiated cells using a medium with GlcNAc, a medium with ManNAc and a medium with Neu5Ac added thereto.
Fig. 3 shows influence of the time for adding GlcNAc, ManNAc and Neu5Ac on expression of a Hcrt gene.
Fig. 4 shows a schedule of induction of neural differentiation by a SFEB/gfCDM method.
Fig. 5 shows expression of a Hcrt gene in neurally differentiated cells with GlcNAc, ManNAc and Neu5Ac added thereto, which were made by a SFEB/gfCDM method.
Fig. 6 shows an outline of culture and differentiation induction of a mouse fetus-derived neurosphere (NSph).
Fig. 7 shows the Hcrt inducing action of ManNAc on a cell differentiated from NSph.
Fig. 8 shows that a Sirt1 inhibitor (EX-527) enhances expression of a Hcrt gene by ManNAc.
Fig. 9 shows that an Ogt inhibitor (BADGP) enhances expression of a Hcrt gene by ManNAc.
Fig. 10 shows that EX-527 and BADGP enhance expression of a Hcrt gene by ManNAc.
Fig. 11 shows a protocol of inducing differentiation into a nerve cell from a mouse ES cell and a human iPS cell using ManNAc and an inhibitor.

### MODES FOR CARRYING OUT THE INVENTION

In the present invention, an orexin neuron refers to a nerve cell which expresses an orexin (hypocretin: Hcrt) gene. The orexin neuron is mainly distributed in the lateral nucleus of hypothalamus in vivo. The orexin neuron is differentiation-induced from a pluripotent stem cell or a neural progenitor cell in vitro by the production method of the present invention described later. Whether a nerve cell is the orexin neuron or not can be confirmed by investigating and identifying the presence or absence of expression of a Hcrt gene in the cell by a known procedure such as a PCR method, in situ hybridization, immunological detection using an antibody (immunostaining, Western blotting, enzyme immunoassay (ELISA)), or further detection of a reaction of the neuron on various stimulations.

Regarding Hcrt, an mRNA and a protein of human Hcrt are published as NCBI Reference Sequence: NM_001524.1, and an mRNA and a protein of mouse Hcrt are published as NCBI Reference Sequence: NM_010410.2.

In the present invention, N-acetyl-D-mannosamine may be an N-acetyl form of D-mannosamine represented by the following formula (I):

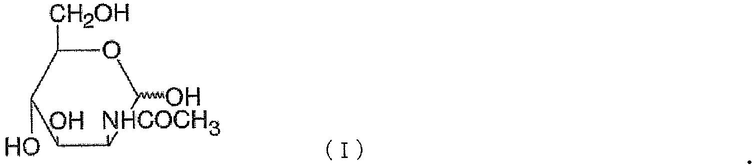

A compound represented by the formula (I) is abbreviated as "ManNAc" in some cases.

In the present invention, N-acetyl-D-mannosamine is not limited to a simple substance represented by the formula (I), and is a concept including a derivative, a precursor or a prodrug thereof, a salt thereof, and a solvate thereof (hereinafter, referred to as "derivative etc." in some cases).

N-acetyl-D-mannosamine may be, for example, a compound represented by the following formula (II): 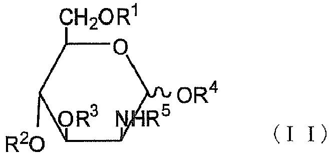 [wherein each of R¹, R², R³, R⁴ and R⁵ independently represents hydrogen (H), R⁶, -C(=O)R⁶, -C(=O)OR⁶, or -C(=O)NR⁶R⁷; R⁶ represents an optionally substituted C₁-C₇ linear or cyclic hydrocarbon; R⁷ represents hydrogen (H), or an optionally substituted C₁-C₇ linear or cyclic hydrocarbon.]

Useful substituents are F, Cl and Br.

Salts of N-acetyl-D-mannosamine include pharmacologically acceptable salts, for example, salts with inorganic acids, salts with organic acids, and salts with basic or acidic amino acids.

Examples of the salts with inorganic acids include salts with hydrochloric acid, hydrobromic acid, nitric acid, sulfuric acid, and phosphoric acid.

Examples of the salts with organic acids include salts with benzoic acid, formic acid, acetic acid, trifluoroacetic acid, fumaric acid, oxalic acid, tartaric acid, maleic acid, citric acid, succinic acid, malic acid, methanesulfonic acid, benzenesulfonic acid, and p-toluenesulfonic acid.

Examples of the salts with basic amino acids include salts with arginine, lysine and ornithine, and examples of the salts with acidic amino acids include salts with aspartic acid and glutamic acid.

Preferred examples of the solvate include hydrates (e.g., monohydrates and dihydrates) and ethanolate.

The N-acetyl-D-mannosamine used may be, a commercially available product, or may be produced by a method known per se. Examples of a method for producing N-acetyl-D-mannosamine represented by the formula (I) include, but are not limited to, a method involving isomerizing N-acetylglucosamine under an alkaline condition (JP-A-HEI-10-182685) and a method involving a reaction of sialic acid as the substrate with N-acetylneuraminic acid lyase (JP-A-2001-78794). The derivative etc. of N-acetyl-D-mannosamine can also be produced by a method known per se using N-acetyl-D-mannosamine represented by the formula (I) as a raw material.

In the present invention, a "Sirtuin inhibitor" refers to a substance which inhibits expression or the activity of Sirtuin (a protein family including Sirt1 to 7). Sirtuin is a factor for regulating metabolism or aging, and Sirt1 to 3, 5 and 6 are known as a deacetylase of a protein including histone (Nature Reviews Molecular Cell Biology, 13, 225-238 (2012)). Examples of the Sirtuin inhibitor include, but are not limited to, a siRNA, a shRNA, an antisense RNA, and an antisense DNA which specifically inhibit expression of a Sirtuin gene, and a low-molecular weight compound which inhibits the activity of a protein deacetylase. Examples of a suitable Sirtuin inhibitor include 6-chloro-2,3,4,9-tetrahydro-1H-carbazole-1-carboxamide (EX-527: SIGMA-ALDRICH) commercially available as a Sirt1 inhibitor.

In the present invention, an "O-linked β-N-acetylglucosamine (O-GlcNAc) transferase inhibitor" refers to a substance which inhibits expression or the activity of O-linked β-N-acetylglucosamine transferase (Ogt). Ogt is an enzyme which catalyzes addition of N-acetylglucosamine to a hydroxy group of serine or threonine (Nature Reviews Cancer, 11, 678-684 (2011)). Examples of the Ogt inhibitor include, but are not limited to, a siRNA which specifically inhibits expression of an Ogt gene and a low-molecular weight compound which inhibits the N-acetylglucosamine addition activity. Examples of a suitable Ogt inhibitor include commercially available benzyl 2-acetamido-2-deoxy-α-D-galactopyranoside (BADGP: SIGMA-ALDRICH).

### <Method for producing orexin neuron>

The present invention provides a method for producing an orexin neuron comprising a step of culturing a pluripotent stem cell or a neural progenitor cell in the presence of N-acetyl-D-mannosamine.

The present invention also provides a method for producing an orexin neuron comprising a step of culturing a pluripotent stem cell or a neural progenitor cell in the presence of N-acetyl-D-mannosamine, and at least one inhibitor selected from the group consisting of a Sirt inhibitor and an Ogt inhibitor.

In the present invention, a "pluripotent stem cell" refers to an undifferentiated cell having a "self-regenerating ability" that it can proliferate while maintaining the undifferentiated state and a "differentiation pluripotency" that it can be differentiated into all or a part of triploblastic systems, particularly a nerve cell. Examples of the pluripotent stem cell include an embryonic stem (ES) cell, a primordial germ stem (EG) cell, a multipotent germ cell (mGS); a somatic stem cell such as a neural stem cell isolated from an adult or fetal brain, a mesenchymal stem cell isolated from bone marrow, blood or an adult tissue, a stromal cell, a fibroblast; and an induced pluripotent stem (iPS) cell.

In the present invention, a "neural progenitor cell" refers to a cell which underwent decision of differentiation into a nerve cell from the "pluripotent stem cell" via a neural progenitor cell formation stage. The neural progenitor cell can be obtained as a fetus-derived neurosphere. Alternatively, the neural progenitor cell can be obtained by culturing a differentiation pluripotent stem cell under a suitable neural differentiation condition, or dedifferentiating a somatic cell by a suitable method, and culturing this under the neural differentiation condition.

The cell that is a subject of the present invention is derived from a mammal such as mouse, rat, pig, rabbit, cat, dog, cow, horse, monkey or human, preferably mouse or human.

More specifically, examples of the embryonic stem cell used in the method of the present invention include an embryonic stem cell of a mammal which is established by culturing an early embryo before implantation (hereinafter, abbreviated as "embryonic stem cell I"), an embryonic stem cell which is established by culturing an early embryo made by transplanting a nucleus of a somatic cell (hereinafter, abbreviated as "embryonic stem cell II"), and an embryonic stem cell in which a gene on a chromosome of an embryonic stem cell of an embryonic stem cell I or II is modified using a genetic engineering technique (hereinafter, abbreviated as "embryonic stem cell III").

More specifically, examples of the embryonic stem cell I include an embryonic stem cell which is established from an inner cell mass constituting an early embryo, an EG cell which is established from a primordial germ cell, a cell isolated from a cell population (e.g. primitive ectoderm) having the multipotency of an early embryo before implantation, and a cell obtained by culturing the cell.

The embryonic stem cell I can be prepared by culturing an early embryo before implantation according to the method described in the literature (Manipulating the Mouse Embryo A Laboratory Manual, Second Edition, Cold Spring Harbor Laboratory Press (1994)).

The embryonic stem cell II can be made using, for example, the method reported by Wilmut et al. (Nature, 385, 810 (1997)), Cibelli et al. (Science, 280, 1256 (1998)), Akira IRITANI et al. (protein nucleic acid enzyme, 44, 892 (1999)), Baguisi et al. (Nature Biotechnology, 17, 456 (1999)), Wakayama et al. (Nature, 394, 369 (1998); Nature Genetics, 22, 127 (1999); Proc. Natl. Acad. Sci. USA, 96, 14984 (1999)), Rideout III et al. (Nature Genetics, 24, 109 (2000)) and the like, as follows.

Development is initiated by using a method of removing a nucleus of a mammalian cell, reprogramming the nucleus (operation of returning the nucleus into a state where development can be repeated again) and injecting the nucleus into an enucleated unfertilized egg of a mammal. By culturing the egg which has initiated development, an egg having a nucleus of another somatic cell which has initiated normal development is obtained.

As a method of reprogramming a nucleus of a somatic cell, a plurality of methods are known. Reprogramming can be performed by inducing a cell cycle into a resting phase state (G0 phase or G1 phase) by, for example, conducting culturing by changing, as a medium in which a cell donating a nucleus is cultured, from a medium containing 5 to 30%, preferably 10% bovine fetal serum (e.g. M2 medium) to an oligotrophic medium containing 0 to 1%, preferably 0.5% bovine fetal serum for 3 to 10 days, preferably for 5 days.

Alternatively, reprogramming can be performed by injecting a nucleus of a cell donating a nucleus into an enucleated unfertilized egg of the same species of a mammal, and culturing the egg for a few hours, preferably for about 1 to 6 hours.

The reprogrammed nucleus can initiate development in an enucleated unfertilized egg. As a method of initiating development of an initialized nucleus in an enucleated unfertilized egg, a plurality of methods are known. By transplanting a nucleus, which has been reprogrammed by inducing a cell cycle into a resting phase state (G0 phase or G1 phase), into an enucleated unfertilized egg of the same species of a mammal by an electrofusion method or the like, an ovum can be activated to initiate development.

Development can be initiated by transplanting a nucleus, which has been reprogrammed by injecting a nucleus into an enucleated unfertilized egg of the same species of a mammal, into an enucleated unfertilized egg of the same species of a mammal again by a method using a micromanipulator or the like, stimulating the ovum with an ovum activating substance (e.g. strontium), and treating the ovum with an inhibitory substance of cell division (e.g. cytochalasin B) to inhibit release of a second polar body. This method is suitable for the case where the mammal is mouse or the like, for example.

Once an egg which has initiated development is obtained, an embryonic stem cell can be obtained using a known method described in, for example, Manipulating the Mouse Embryo A Laboratory Manual, Second Edition, Cold Spring Harbor Laboratory Press (1994); Gene Targeting, A Practical Approach, IRL Press at Oxford University Press (1993); Biomanual Series 8 Gene Targeting, Making of Mutant Mouse using ES Cell, Yodosha Co., Ltd. (1995).

The embryonic stem cell III can be made by, for example, using a homologous recombination technique. Examples of a gene on a chromosome to be modified upon preparation of the embryonic stem cell III include a gene of a histocompatibility antigen and a gene associated with a disease based on a disorder of a neural cell. Modification of a target gene on a chromosome can be performed using the method described in, for example, Manipulating the Mouse Embryo A Laboratory Manual, Second Edition, Cold Spring Harbor Laboratory Press(1994); Gene Targeting, A Practical Approach, IRL Press at Oxford University Press (1993); Biomanual Series 8 Gene Targeting, Making of Mutant Mouse using ES Cell, Yodosha Co., Ltd. (1995).

Specifically, for example, a genome gene of a target gene to be modified (e.g. a gene of a histocompatibility antigen or a disease-associated gene) is isolated, and the isolated genome gene is used to make a target vector for homologously recombining a target gene. The obtained target vector is introduced into an embryonic stem cell, and a cell which has caused homologous recombination between the target gene and the target vector is selected, thereby, an embryonic stem cell in which a gene on a chromosome has been modified can be made.

The embryonic stem cell can be obtained from prescribed institutions, or a commercially available product can also be purchased. For example, KhES-1, KhES-2 and KhES-3 which are human embryonic stem cells can be obtained from Institute for Frontier Medical Sciences, Kyoto University.

In the present invention, a "somatic stem cell" refers to an undifferentiated cell having the "differentiation pluripotency" that it can be differentiated into a neural cell of an ectoderm lineage, and is not derived from a germ cell. Examples of the somatic stem cell include a neural stem cell, a mesenchymal stem cell, a stromal cell, a fibroblast, and an adipocyte precursor cell.

Mouse and human induced pluripotent stem cells (iPS cells) can be established according to WO 2004/092357 and WO 2007/069666. Alternatively, it is also possible to make an iPS cell by three factors except for a c-Myc gene (representative examples of which are Oct3/4, Klf4, and Sox2) (Takahashi, K. and Yamanaka, S., Cell, 126: 663-676 (2006)), or to induce an iPS cell using a plasmid or episomal vector without incorporation of an reprogramming factor into a genome (Okita, K. et al., Science, 322: 949-953 (2008); Yu, J. et al., Science, 324: 797-801 (2009)). The iPS cell can be established from, for example, a somatic cell such as a skin fibroblast according to the method described in the above literature.

The iPS cell can be obtained from prescribed institutions. For example, a 201B7 strain which is a human iPS cell can be obtained from Riken BioResource Center, RIKEN, Japan.

It is desirable to use, as the neural progenitor cell, a fetus-derived neural mass (derived from an endbrain, also referred to as neurosphere). In the case of a mouse fetus, it is desirable to remove the endbrain at an embryonic age of 14.5 days. Culture of the neural progenitor cell can be performed in accordance with the method described in Ciccolini F, Svendsen CN. Fibroblast growth factor 2 (FGF-2) promotes acquisition of epidermal growth factor (EGF) responsiveness in mouse striatal precursor cells: identification of neural progenitors responding to both EGF and FGF-2. (1998), J Neurosci. 18(19): 7869-80, and a detailed culture method is described in Examples described later.

A medium used in the production method of the present invention can be prepared using, as a basal medium, a medium which is used in culture of an animal cell. The basal medium is not particularly limited as far as it is a medium which can be used in culture of an animal cell, such as a BME medium, a BGJb medium, a CMRL 1066 medium, a Glasgow MEM medium, an Improved MEM Zinc Option medium, an IMDM medium, a Medium 199 medium, an Eagle MEM medium, an αMEM medium, a DMEM medium, a DMEM/F12 medium, a Ham medium, a RPMI 1640 medium, a Fischer's medium, and a mixed medium of them.

The medium used in the production method of the present invention can be a serum-containing medium or a serum-free medium. Herein, the serum-free medium means a medium not containing non-adjusted or unpurified serum, and a medium containing a purified blood-derived component or an animal tissue-derived component (e.g. growth factor) corresponds to the serum-free medium. Examples of such a serum-free medium include a serum-free medium to which an appropriate amount (e.g. 1 to 20%) of commercially available KNOCKOUT™ SR (KSR) has been added, a serum-free medium to which insulin and transferrin have been added (e.g. CHO-S-SFM II (manufactured by GIBCO BRL), Hybridoma-SFM (manufactured by GIBCO BRL), eRDF Dry Powered Media (manufactured by GIBCO BRL), UltraCULTURE™ (manufactured by BioWhittaker), UltraDOMA™ (manufactured by BioWhittaker), UltraCHO™ (manufactured by BioWhittaker), UltraMDCK™ (manufactured by BioWhittaker), an ITPSG medium (Cytotechnology, 5, S17 (1991)), an ITSFn medium (Proc. Natl. Acad. Sci. USA, 77, 457 (1980)), and an mN3 medium (Mech. Dev. 59, 89 (1996)), and a medium to which a factor derived from a cell has been added (e.g. a medium to which the culture supernatant of a pluripotent teratocarcinoma cell PSA1 has been added (Proc. Natl. Acad. Sci. USA, 78, 7634 (1981)).

Furthermore, the medium used in the production method of the present invention may optionally contain other components, for example, an amino acid, pyruvic acid, 2-mercaptoethanol, a cytokine, and a growth factor in an appropriate concentration.

A culture vessel used in a culturing step of the production method of the present invention is not particularly limited as far as it is intended for cell culturing, and examples thereof include a flask, a flask for tissue culturing, a dish, a petri dish, a dish for tissue culturing, a multidish, a microplate, a microwell plate, a multiplate, a multiwell plate, a chamber slide, a schale, a tube, a tray, a culturing bag, and a roller bottle.

A medium and a culture vessel are appropriately selected from the aforementioned media and culture vessels according to the kind of the cell and an object, and culture of a pluripotent stem cell or a neural progenitor cell embryonic stem cell is initiated under a condition where the cell is differentiated into a nerve cell in vitro. Examples of a general culturing condition include a method of culturing for around 1 to 20 days in an incubator aerated with 2 to 10% CO₂ at 32 to 40°C.

In the case of addition of N-acetyl-D-mannosamine alone, it can be added to a medium from the starting day of culture (0th day of differentiation) until completion of culturing. Alternatively, an orexin neuron can also be induced by adding N-acetyl-D-mannosamine on 4th to 7th day of differentiation.

In the case of combined addition of N-acetyl-D-mannosamine and at least one inhibitor selected from the group consisting of a Sirt inhibitor and an Ogt inhibitor, N-acetyl-D-mannosamine can be added to a medium from the starting day of culture (0th day of differentiation) until completion of culturing. Alternatively, N-acetyl-D-mannosamine can also be added to a medium on 4th to 7th day of differentiation. The Sirt inhibitor and/or the Ogt inhibitor may be added from the 0th day of differentiation, but a protocol of adding the inhibitor to a medium on 4th to 7th day of differentiation is recommended.

It is recommended that the concentration of N-acetyl-D-mannosamine in a medium is 0.001 to 5 mM, preferably 0.01 to 1 mM. The concentration of the Sirt inhibitor in a medium is appropriately determined according to the kind of the inhibitor, and when EX-527 is used, 1 to 200 nM, preferably 5 to 50 nM is recommended. The concentration of the Ogt inhibitor in a medium is appropriately determined according to the kind of the inhibitor, and when BADGP is used, 1 to 50 mM, preferably 2 to 10 mM is recommended.

In the production method of the present invention, in order to more improve the efficiency of differentiation from a pluripotent stem cell or a neural progenitor cell into a neural cell, a known substance inducing differentiation into a neural cell can be used together. Examples of such a differentiation inducing substance include NGF, BDNF, NT3, retinoic acid, FGF, a BMP inhibiting factor, IGF and CNTF. The concentration of the differentiation inducing substance in a medium is appropriately determined according to the kind of the differentiation inducing substance.

In one embodiment, differentiation into an orexin neuron is induced by pre-culturing a stem cell or a progenitor cell for 4 to 7 days and, thereafter, adding N-acetyl-D-mannosamine alone to a medium in the presence of the substance inducing differentiation into a neural cell, if necessary.

In another embodiment, differentiation induction into an orexin neuron is enhanced by adding N-acetyl-D-mannosamine to a medium on the day when culture of a stem cell or a progenitor cell is initiated (0th day), and thereafter, adding at least one inhibitor selected from the group consisting of a Sirt inhibitor and an Ogt inhibitor to the medium on 4th to 7th days.

By the production method of the present invention, an orexin neuron can be efficiently induced in vitro, and a cultured orexin neuron can be provided.

### <Screening method>

The present invention provides a method for screening for a drug which acts on regulation of wakefulness-sleep, using an orexin neuron obtainable by the production method of the present invention.

In the screening method of the present invention, culture of the orexin neuron obtained as described above is continued under a condition where the orexin neuron is alive in vitro, a test substance is added to a medium, and proliferation of the orexin neuron is observed for a prescribed term.

Herein, the test substance may be any known substance and novel substance, and examples thereof include a nucleic acid, a saccharide, a fat, a protein, a peptide, an organic low-molecular weight compound, a compound library made using a combinatorial chemistry technique, a random peptide library made by solid phase synthesis or a phage display method, and a natural component derived from a microorganism, an animal, a plant, a marine organism or the like.

A method of culturing (maintaining) the orexin neuron in vitro is as described above in the production method of the present invention. In the present step, it is desirable to use a medium free of N-acetyl-D-mannosamine, a Sirt inhibitor and an Ogt inhibitor.

The presence or absence of proliferation of the orexin neuron can be confirmed by investigating the number and the distribution state of orexin neurons in a test substance-free group and a test substance-containing group by, for example, an in situ hybridization method using expression of a Hcrt gene as an index. Alternatively, a staining method using an antibody such as a fluorescent immunostaining method using a fluorescent antibody, a method of detecting or measuring orexin which has been released into a culture solution by treatment under suitable stimulation, or orexin which has been accumulated in a cell by an immunological procedure such as ELISA or Western blotting, and a method of reacting the culture supernatant or the cell extract with an orexin-sensitive neuron, a cell which expresses a receptor by introducing an orexin receptor gene such as that used upon isolation of orexin, or a kidney cell or a testis cell which expresses an orexin receptor, and detecting the reaction by an appropriate method can be used.

When proliferation of the orexin neuron is significantly promoted in a test substance-containing group as compared with a test substance-free group, the test substance is a candidate of a drug which acts on regulation of wakefulness-sleep via proliferation (induction) of the orexin neuron. The selected test substance includes not only a so-called agonist or antagonist of Hcrt, but also a substance which can vary the expression amount of these proteins from the nature of the present screening method.

A drug selected by the screening method of the present invention acts on regulation of wakefulness-sleep via proliferation (induction) or functional potentiation of the orexin neuron, and is expected as a candidate therapeutic for a brain dysfunction such as reduction in cognitive or learning capacity, a REM sleep disorder exhibiting fragmentation of REM sleep, particularly a problem of wakefulness-sleep caused by aging or a neurogenic disease accompanied with aging, and further, narcolepsy.

### <An agent and a kit for inducing an orexin neuron>

The present invention provides an agent for inducing an orexin neuron from a neural progenitor cell or a nerve cell containing N-acetyl-D-mannosamine as an active ingredient.

The present invention also provides a kit for inducing an orexin neuron from a neural progenitor cell or a nerve cell, in which reagents containing
(1) N-acetyl-D-mannosamine, and
(2) at least one inhibitor selected from the group consisting of a Sirtuin 1 inhibitor and an O-linked β-N-acetylglucosamine transferase inhibitor as active ingredients are accommodated in separate containers.

An active ingredient contained in the inducing agent and the kit of the present invention is as described above in the production method of the present invention. As shown in Examples described later, the orexin neuron inducing action of N-acetyl-D-mannosamine is enhanced by combined use with a Sirt inhibitor or an Ogt inhibitor, and is further enhanced by combined use with a Sirt inhibitor and an Ogt inhibitor.

A reagent contained in the inducing agent and the kit of the present invention may contain an optional ingredient to be added to a medium. Examples of the optional ingredient include, but are not limited to, a solvent, a buffer, and an antiseptic.

### EXAMPLES

The present invention will be explained more specifically by way of examples. The following shows representative examples, and the present invention is not limited to them. However, a variety of applications are possible within the bounds of not departing from the technical idea of the present invention.

### Example 1: Induction of expression of an orexin gene (Hcrt) with ManNAc using a system of induction of neural differentiation from an ES cell

### Analysis using a stromal cell-derived inducing activity (SDIA) differentiation culture system

### Differentiation culturing

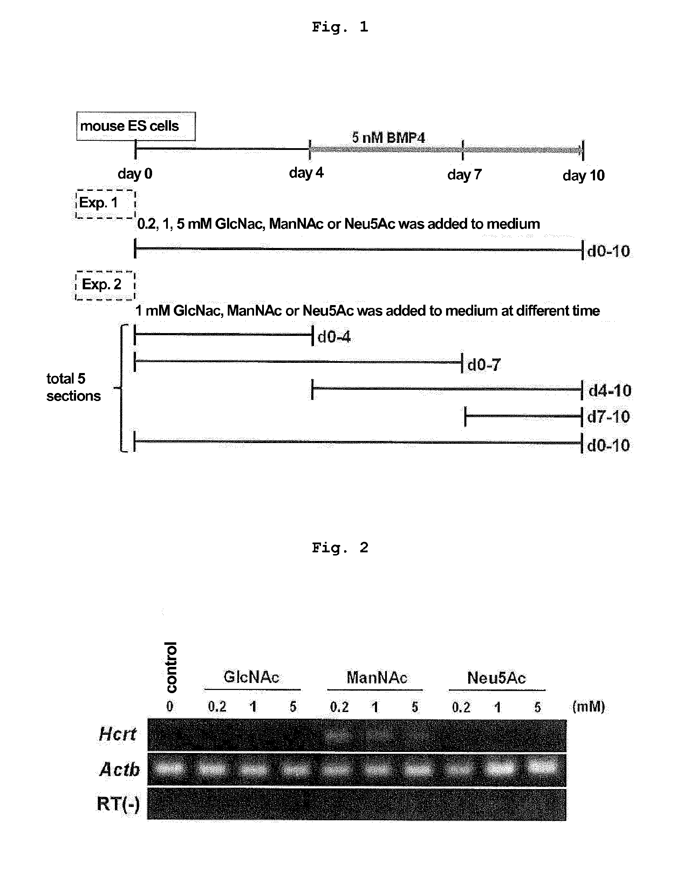

Mouse ES cells (J1 strain) which had been maintained in a medium for an ES cell [DMEM (Wako) added with 15% FBS (Biowest), 1 mM sodium pyruvate (Invitrogen), 100 mM β-mercaptoethanol (Invitrogen), 2 mM L-glutamine (Invitrogen), 1 mM non essential amino acid (Invitrogen), 50 U/ml penicillin/50 µg/ml streptomycin (Invitrogen), 2000 U leukemia inhibitory factor (Chemicon, CA)] were seeded on a 10 cm culture dish so that the cell count became 1 × 10⁵ cells per culture dish on which PA6 stromal cells had been seeded in advance, and differentiation induction was performed in a medium for neural differentiation [Glasgow MEM (Invitrogen) added with 10% KSR (Invitrogen), 100 mM β-mercaptoethanol (Invitrogen), 1 mM non essential amino acid (Invitrogen), 50 U/ml penicillin/50 µg/ml streptomycin (Invitrogen)] for 10 days. On 4th and 7th days of differentiation culturing, the medium was exchanged. In addition, from 4th day of differentiation culturing, 5 nM recombinant human BMP4 (Wako) was added (Fig. 1). On 10th day of differentiation culturing, cells were collected and pelletized by a centrifugation operation to remove the culture solution. Thereafter, the pellets were immediately frozen in liquid nitrogen and stored at -80°C until use.

### References

Mizuseki K, Sakamoto T, Watanabe K, Muguruma K, Ikeya M, Nishiyama A, Arakawa A, Suemori H, Nakatsuji N, Kawasaki H, Murakami F, Sasai Y. Generation of neural crest-derived peripheral neurons and floor plate cells from mouse and primate embryonic stem cells. (2003) Proc. Natl. Acad. Sci. U S A. 100(10):5828-33.

### Expression analysis by an RT-PCR method

The total RNA was extracted from the collected cells using RNeasy plus Mini Kit (QIAGEN). Then, a reverse transcription reaction was performed in a reaction liquid containing 1 µg of the total RNA, and Oligo (dT) using SuperScript III First Strand Synthesis System (Invitrogen) to synthesize a cDNA.

A PCR reaction was performed on a 10 µl scale, 1 U of LA-Taq DNA Polymerase (Takara) was added to 0.5 µl of a cDNA, 5 µl of 2 × GC Buffer I, each 200 µM of dNTP, 1.5 mM MgCl₂, and each primer having a final concentration of 0.2 µM, thermal denaturation was performed at 95°C for 3 minutes, and the reaction was performed under the condition of (30 seconds at 95°C, 30 seconds at 55°C, and 30 seconds at 72°C) × 35 cycles (20 cycles in the case of Actb). The primers used are shown below.
Hcrt Forward; 5'- CTCCAGGCACCATGAACTTT -3' (SEQ ID NO: 1)
Hcrt Reverse; 5'- AGTTCGTAGAGACGGCAGGA -3' (SEQ ID NO: 2)
Actb Forward; 5'- TTCTACAATGAGCTGCGTGTGG -3' (SEQ ID NO: 3)
Actb Reverse; 5'- ATGGCTGGGGTGTTGAAGGT -3' (SEQ ID NO: 4)

Each PCR product was electrophoresed on a 2% agarose gel, stained with ethidium bromide, and a band was observed by UV irradiation.

### Results

### (Experiment 1, Fig. 2)

From the initiation of differentiation culturing, GlcNAc (N-acetyl-D-glucosamine), ManNAc and Neu5Ac (N-acetylneuraminic acid) were added to a neural differentiation medium so that the concentration became 0.2 and 1.0 mM, and cells were collected on 10th day of differentiation culturing and subjected to RT-PCR. Expression of an orexin gene (Hcrt) was analyzed, and as a result, expression of Hcrt was recognized only in a ManNAc-added section. In addition, a similar effect was obtained also in a human-derived pluripotent stem cell.

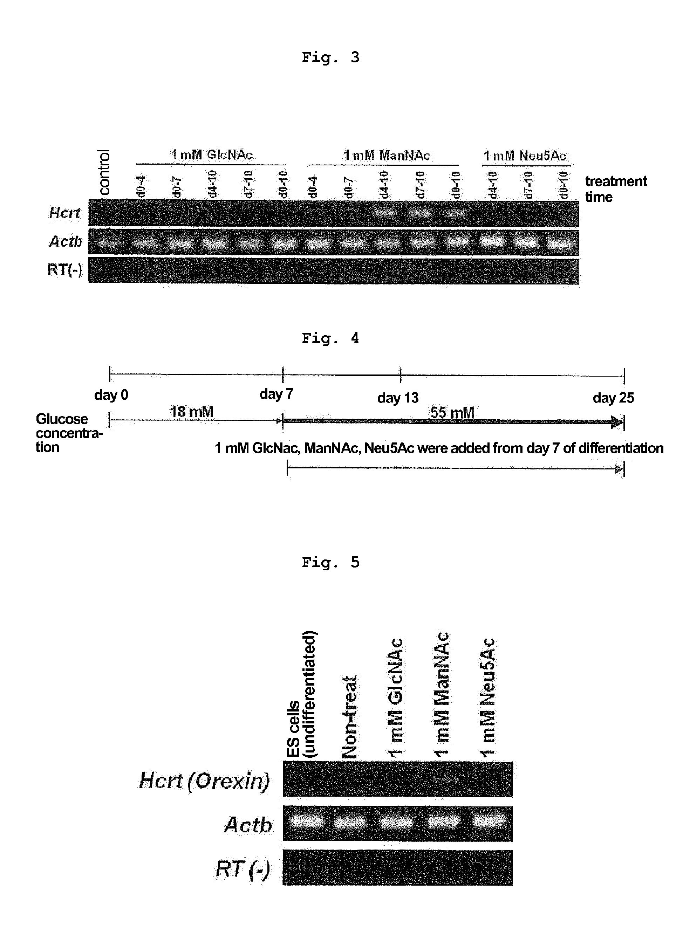

### (Experiment 2, Fig. 3)

Then, GlcNAc, ManNAc and Neu5Ac were added at the concentration of 1.0 mM by changing the time for adding them, and neural differentiation culturing was performed. As a result of analysis by RT-PCR, expression of Hcrt was observed only in a ManNAc-added section at any addition time. In addition, in the 1.0 mM ManNAc-added section, high expression was observed in the section in which ManNAc was added to a medium on and after 7th day of differentiation (ManNAc was added on 4th to 10th days, 7th to 10th days and 0th to 10th days) as compared with other two sections (ManNAc was added on 0th to 4th days and 0th to 7th days).

### Example 2: Analysis using a SFEB/gfCDM* differentiation culture system

*: abbreviation of serum-free culture of embryoid body-like aggregates/growth factor-free chemically defined medium

### Differentiation culturing

Mouse ES cells (J1 strain) which had been maintained in a medium for an ES cell were seeded on a 96-well low adhering round-bottom culture dish (Nunc) so that the cell count became 10,000 cells per well, and cultured with gfCDM [IMDM/F12 (Invitrogen) added with 5 mg/ml BSA (SIGMA), 450 µM Monothioglycerol (Wako), 1x chemically defined lipid concentrate (Invitrogen), 50 U/ml penicillin/50 µg/ml streptomycin] for 7 days. The cells were cultured in 150 µl of a medium per well. On 7th day of differentiation culturing, the medium was exchanged with a DFK medium [DMEM/F12 (Invitrogen) added with 7 g/l glucose, 10% KSR, 50 U/ml penicillin/50 µg/ml streptomycin], and on 10th day, half the amount of the medium was exchanged with a DFNB medium [7 g/l glucose, 1x N2 (Wako), 1xB27 (Invitrogen)] containing 10 ng/ml CNTF. On 13th day of culturing, cells were detached with 0.05% trypsin/EDTA, seeded on a culture dish (Falcon) which had been treated with poly-D-lysine/laminin so that the cell count became 4.8 × 10⁶ cells per 10 cm culture dish, and cultured in a DFNB medium containing 10% FBS, 10 ng/ml CNTF, 50 ng/ml BDNF and 50 ng/ml NT3. The medium was exchanged every 3 days. On 25th day of differentiation culturing, cells were collected and pelletized by a centrifugation operation to remove the culture solution. Thereafter, the pellets were immediately frozen in liquid nitrogen, and stored at -80°C until use.

### References

Wataya T, Ando S, Muguruma K, Ikeda H, Watanabe K, Eiraku M, Kawada M, Takahashi J, Hashimoto N, Sasai Y. Minimization of exogenous signals in ES cell culture induces rostral hypothalamic differentiation. (2008) Proc. Natl. Acad. Sci. USA., 105(33):11796-801.

### Expression analysis by an RT-PCR method

The analysis was performed by the same method as that of Example 1.

### Results (Figs. 4 and 5)

From 7th day of differentiation culturing, GlcNac, ManNAc and Neu5Ac were added to a medium at the concentration of 1.0 mM, and neural differentiation induction was performed. As a result, expression of Hcrt was recognized in ManNAc and Neu5Ac-added sections. In addition, high expression of Hcrt was observed in a ManNAc-added section as compared with a Neu5Ac-added section.

### Example 3: Orexin inducing action of ManNAc on a mouse fetus-derived neurosphere (NSph)

### Culture of an endbrain-derived neurosphere (NSph) and differentiation induction into a neuron

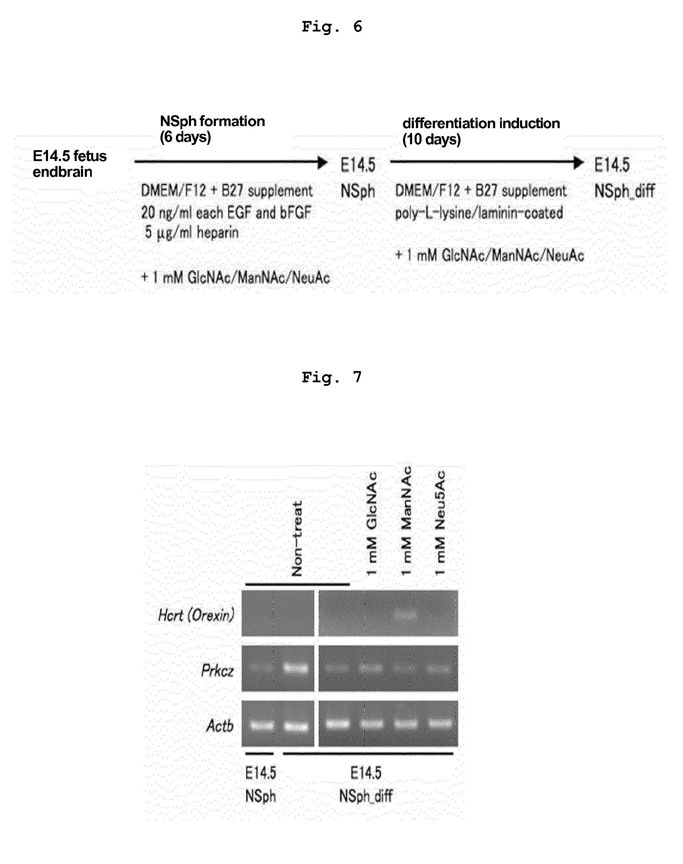

A culture and differentiation induction experiment was performed based on the method of Ciccolini and Svendsen (1998, J Neurosci. 18(19): 7869-80). An outline is shown in Fig. 6. An endbrain was removed from a C57BL/6N mouse fetus having an embryonic age of 14.5 days in ice-cold PBS/0.6% glucose. Dispersion into a single cell was performed by pipetting, and cells were suspended in a medium for NSph [DMEM/F12 (Wako) containing 1xB27 (Invitrogen), 20 ng/ml EGF (Sigma), 20 ng/ml FGF-basic (PEPROTECH), 5 µg/ml heparin (Sigma)] and seeded on a 10 cm petri dish in an amount of 2 × 10⁶ cells/10 ml. Half the amount of the medium was exchanged 3 days after initiation of culturing, and culturing was performed for another 3 days (total 6 days).

NSph was recovered 6 days after initiation of culturing, and cells were dispersed by pipetting in PBS/0.6% glucose. Cells were collected by centrifugation and suspended in a medium for differentiation induction (not containing EGF, FGF-basic and heparin of a medium for NSph). Cells were seeded in an amount of 4.5 × 10⁵ cells/7.5 ml on a 6 cm dish which had been coated with poly-L-lysine/laminin (both Sigma) in advance, and differentiation was induced. Half the amount of the medium was exchanged 5 days after initiation of differentiation induction, and culturing was performed for another 5 days (total 10 days). The cells were washed with PBS and then lysed with 0.5 ml TRIzol Reagent (Invitrogen) to be recovered in a 1.5 ml microtube, and stored at -80°C until use.

### Expression analysis by an RT-PCR method

The total RNA was extracted from the cells which had been lysed with TRIzol Reagent. Using SuperScript III First Strand Synthesis System (Invitrogen), a reverse transcription reaction was performed in 10 µl of a reaction liquid containing 90 ng of the total RNA and Oligo (dT) to synthesize a cDNA. The reaction liquid was diluted 5-fold with 10 mM Tris-HCl (pH 8.0)/0.1 mM EDTA, and used in PCR.

A reaction liquid (20 µl) containing 1 × ImmoBuffer, 1.5 mM MgCl₂, 0.2 mM each dNTP, 0.2 µM forward primer, 0.2 µM reverse primer, 1 U of BIOTAQ HS Polymerase (BIOLINE) and 2 µl of a cDNA was prepared, and after one cycle of 7 minutes at 95°C, PCR was performed under the condition of 24 (Actb)/32 (Prkcz)/36 (Hcrt) cycles of 30 seconds at 94°C, 30 seconds at 62°C, and 30 seconds at 72°C. The primers used are shown below. Actb forward; 5'- GACAACGGCTCCGGCATGTGCAAAG -3' (SEQ ID NO: 5) Actb reverse; 5'- TTCACGGTTGGCCTTAGGGTTCAG -3' (SEQ ID NO: 6) Prkcz forward; 5'- ATGTCTGCTCCTCCAGCAGT -3' (SEQ ID NO: 7) Prkcz reverse; 5'- ATATCCTTTCGCTGCACTGG -3' (SEQ ID NO: 8) Hcrt forward; 5'- CTCCAGGCACCATGAACTTT -3' (SEQ ID NO: 1) Hcrt reverse; 5'- AGTTCGTAGAGACGGCAGGA -3' (SEQ ID NO: 2)

The PCR product was electrophoresed on a 2% agarose gel, stained with ethidium bromide, and a band was observed by UV irradiation.

### Results

At the time when culturing of NSph was initiated, GlcNAc, ManNAc or NeuAc was added so that the concentration became 1 mM, and culturing was performed for 6 days. Subsequently, differentiation induction was performed for 10 days with the added GlcNAc, ManNAc or NeuAc left as it is, and cells were collected (Fig. 6).

Expression of Hcrt analyzed by RT-PCR was detected only in a 1.0 mM ManNAc-added culture (Fig. 7).

### Example 4: Induction of expression of an orexin gene (Hcrt) with ManNAc as well as a Sirt inhibitor and an Ogt inhibitor using a system of induction of neural differentiation from a mouse ES cell

### Differentiation culturing

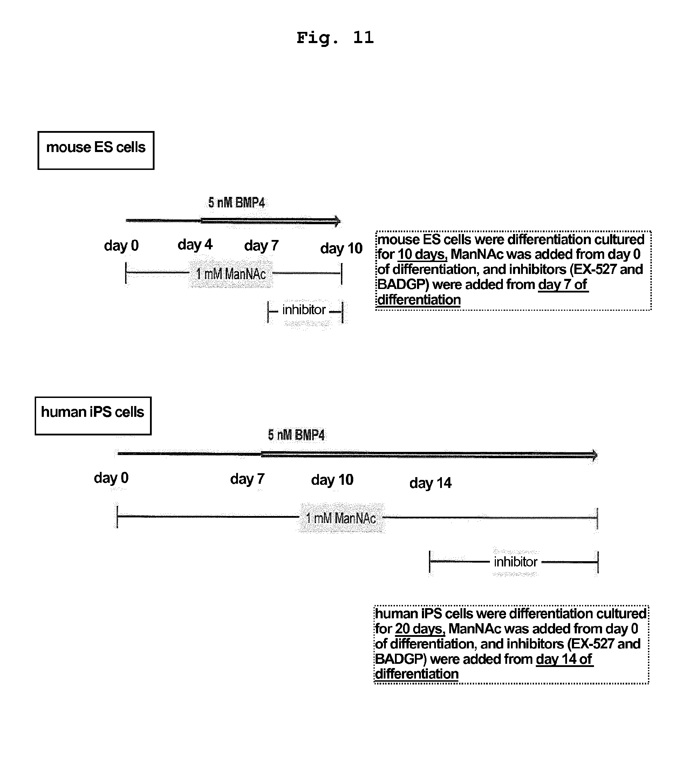

According to the same manner as that described in Example 1, mouse ES cells (J1 strain) were cultured for 10 days for differentiation induction into a nerve cell. A protocol of inducing differentiation into a nerve cell is shown in the upper column of Fig. 11. From the initiation of differentiation culturing (0th day), ManNAc was added to a neural differentiation medium so that the concentration became 1.0 mM, and the medium was exchanged on 4th and 7th days of differentiation culturing. In addition, from 4th day of differentiation culturing, 5 nM recombinant human BMP4 (Wako) was added. On 7th day of differentiation culturing, EX-527 (SIGMA-ALDRICH) was added to a neural differentiation medium so that the concentration became 50 nM or BADGP (SIGMA-ALDRICH) was added to a neural differentiation medium so that the concentration became 1 or 5 mM, and on 10th day of differentiation culturing, cells were collected and subjected to RT-PCR. The conditions of RT-PCR are the same as those of Example 1.

### Results

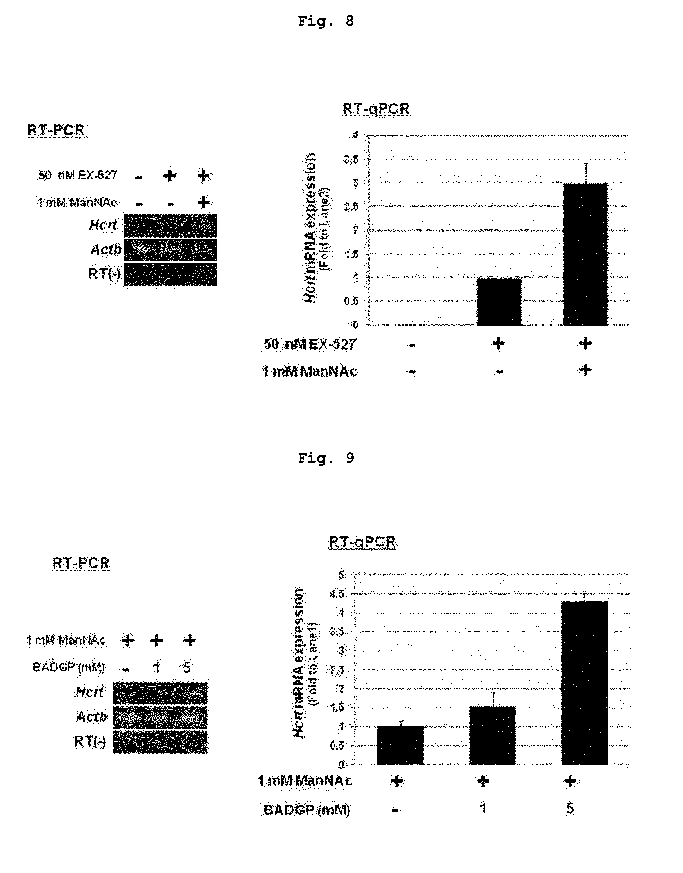

### (Experiment 3, Fig. 8)

From the initiation of differentiation culturing, ManNAc was added to a neural differentiation medium so that the concentration became 1.0 mM, on 7th day of differentiation culturing, EX-527 (SIGMA-ALDRICH) was added to a neural differentiation medium so that the concentration became 50 nM, and on 10th day of differentiation culturing, cells were collected and subjected to RT-PCR. Expression of an orexin gene (Hcrt) was analyzed, and as a result, expression of Hcrt was recognized in an EX-527-added section. It was revealed that expression of Hcrt is enhanced by combined use of ManNAc and EX-527.

### (Experiment 4, Fig. 9)

Then, from the initiation of differentiation culturing, ManNAc was added to a neural differentiation medium so that the concentration became 1.0 mM, on 7th day of differentiation culturing, BADGP (SIGMA-ALDRICH) was added to a neural differentiation medium so that the concentration became 1 mM or 5 mM, and on 10th day of differentiation culturing, cells were collected and subjected to RT-PCR. Expression of an orexin gene (Hcrt) was analyzed, and as a result, it was revealed that expression of Hcrt which had been recognized in a ManNAc-added section is enhanced by combined use of ManNAc and BADGP. A tendency that expression of Hcrt is increased dependent on the concentration of BADGP was observed.

### Example 5: Induction of expression of an orexin gene (Hcrt) with ManNAc as well as a Sirt inhibitor and an Ogt inhibitor using a system of induction of neural differentiation from a human iPS cell

### Differentiation culturing

A human iPS cell strain 201B7 (HSP0001) was obtained from Riken BioResource Center via National BioResource Project (MEXT, Japan). The human iPS cell was maintained on a feeder layer of a mitomycin C-treated STO/Neo resistant/LIF (SNL) feeder cell in a primate ES medium (ReproCELL) complemented with 5 ng/mL recombinant human bFGF (Wako). The human iPS cell was co-cultured with PA6 for 20 days, for differentiation induction into a nerve with a SIDA and BMP4 system. From 7th day, 5 nM BMP4 was complemented. A detailed protocol of differentiation induction into a nerve cell is shown in the lower column of Fig. 11. From the initiation of differentiation culturing (0th day), ManNAc was added to a neural differentiation medium so that the concentration became 1.0 mM, and on 7th, 10th and 14th days of differentiation culturing, the medium was exchanged. In addition, from 7th day of differentiation culturing, 5 nM recombinant human BMP4 (Wako) was added. On 14th day of differentiation culturing, EX-527 (SIGMA-ALDRICH) was added to a neural differentiation medium so that the concentration became 50 nM, or BADGP (SIGMA-ALDRICH) was added to a neural differentiation medium so that the concentration became 5 mM, and on 20th day of differentiation culturing, cells were collected and subjected to RT-PCR. The conditions of RT-PCR are the same as those of Example 1.

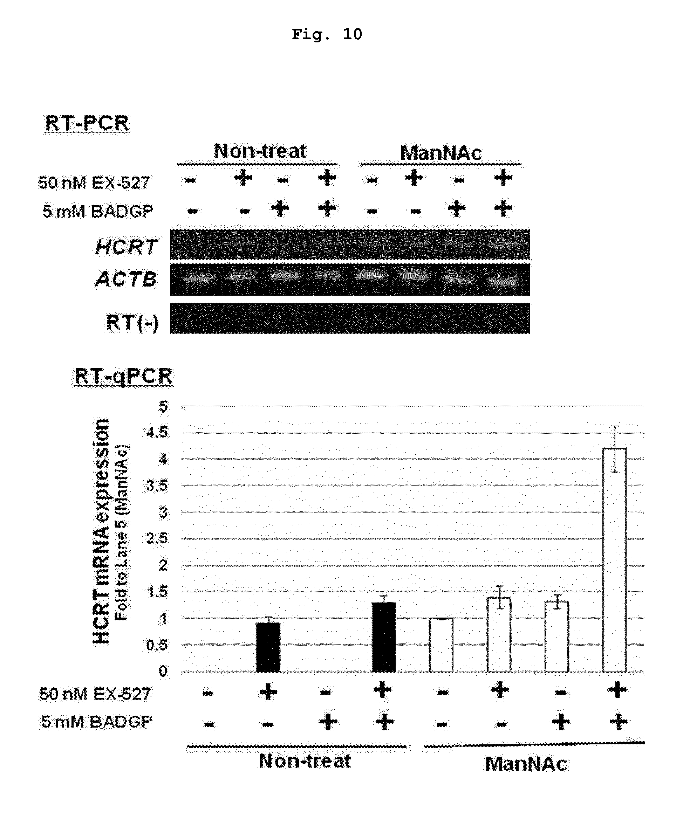

### Results (Fig. 10)

Expression of an orexin gene (Hcrt) was analyzed by RT-PCR, and as a result, it was revealed that expression of Hcrt which had been recognized in a ManNAc-added section and an EX-527-added section to the same extent is enhanced by combined use of ManNAc and EX-527, ManNAc and BADGP, or ManNAc, EX-527 and BADGP. Expression of Hcrt was most enhanced by combined use of three kinds of ManNAc, EX-527 and BADGP.

### Example 6: Analysis of an orexin-positive cell in a neurally differentiated colony which has been differentiation-induced from a human iPS cell

Cells which had been differentiation-induced on a 4-well culture plate by the method described in Example 5 were fixed with a 4% paraformalin solution, and cell membrane permeability treatment was performed with a 0.2% Triton-X100 solution. The cells were treated with a 5% bovine serum albumin solution, followed by treatment with a primary antibody (anti-human orexin antibody, anti-human tubulin III antibody) at 4°C overnight. After a reaction with a secondary labeled antibody (Alex-Fluor 488-labeled donkey anti-goat antibody, Alexa-Fluor 594-labeled rabbit anti-mouse antibody), a nuclear DNA was stained with DAPI. Fluorescence was observed with a fluorescent microscope, and the ratio of orexin-positive cells in a neurally differentiated colony, in which a reaction with an orexin antibody had been recognized, was measured.

As a result, 4.9% of cells became orexin-positive in a ManNAc treatment section, 6.1% of cells became orexin-positive in a ManNAc and EX-527 treatment section, 7.0% of cells became orexin-positive in a ManNAc and BADGP treatment section, and 18.7% of cells became orexin-positive in a ManNAc, EX-527 and BADGP simultaneous treatment section. It was revealed that differentiation of an orexin neuron was remarkably enhanced by a combination of ManNAc and a Sirtuin inhibitor and/or an OGT inhibitor.

### INDUSTRIAL APPLICABILITY

According to the present invention, there are provided medicines, foods and the like containing ManNAc as an active ingredient. By ingestion or consumption of medicines or foods of the present invention, deterioration in the brain function accompanied with aging can be prevented, improved or treated.

This application is based on a provisional US patent application No. 61/540601 filed in US (filing date: September 29, 2011), the contents of which are incorporated in full herein.

## Claims

1. A method for producing an orexin neuron, comprising a step of culturing a pluripotent stem cell or a neural progenitor cell in the presence of N-acetyl-D-mannosamine.

2. The method according to claim 1, wherein the pluripotent stem cell is an embryonic stem cell, a somatic stem cell or an induced pluripotent stem cell.

3. The method according to claim 1, wherein the neural progenitor cell is a fetus-derived neurosphere.

4. An orexin neuron, which is obtainable by the method according to any one of claims 1 to 3.

5. A method for screening for a drug which acts on regulation of wakefulness-sleep, comprising using the orexin neuron according to claim 4.

6. The screening method according to claim 5, wherein the drug is a therapeutic agent for narcolepsy.

7. A method for inducing an orexin neuron, comprising a step of administering an effective amount of N-acetyl-D-mannosamine to a subject in need thereof, or having an effective amount of N-acetyl-D-mannosamine taken by a subject in need thereof.

8. An agent for inducing an orexin neuron from a neural progenitor cell or a nerve cell, comprising N-acetyl-D-mannosamine as an active ingredient.

9. A method for producing an orexin neuron, comprising a step of culturing a pluripotent stem cell or a neural progenitor cell in the presence of N-acetyl-D-mannosamine, and at least one inhibitor selected from the group consisting of a Sirtuin inhibitor and an O-linked β-N-acetylglucosamine transferase inhibitor.

10. The method according to claim 9, wherein the pluripotent stem cell is an embryonic stem cell, a somatic stem cell or an induced pluripotent stem cell.

11. The method according to claim 9, wherein the neural progenitor cell is a fetus-derived neurosphere.

12. An orexin neuron, which is obtainable by the method according to any one of claims 9 to 11.

13. A method for screening for a drug which acts on regulation of wakefulness-sleep, comprising using the orexin neuron according to claim 12.

14. The screening method according to claim 13, wherein the drug is a therapeutic agent for narcolepsy.

15. A kit for inducing an orexin neuron from a neural progenitor cell or a nerve cell, comprising
(1) N-acetyl-D-mannosamine, and
(2) at least one inhibitor selected from the group consisting of a Sirtuin 1 inhibitor and an O-linked β-N-acetylglucosamine transferase inhibitor as active ingredients in separate containers.


SEQUENCE LISTING

<110>  The University of Tokyo

<120>  Methods for Inducing Orexin Neurons

<130>  N402839-EP

<140>  PCT/JP 12/075137
<141>  2012-09-18

<150>  US 61/540,601
<151>  2011-09-29

<160>  8

<170>  PatentIn version 3.5

<210>  1
<211>  20
<212>  DNA
<213>  Artificial Sequence

<220>
<223>  Hcrt primer forward

<400>  1
ctccaggcac catgaacttt                                                   20


<210>  2
<211>  20
<212>  DNA
<213>  Artificial Sequence

<220>
<223>  Hcrt primer reverse

<400>  2
agttcgtaga gacggcagga                                                   20


<210>  3
<211>  22
<212>  DNA
<213>  Artificial Sequence

<220>
<223>  Actb primer forward

<400>  3
ttctacaatg agctgcgtgt gg                                                22


<210>  4
<211>  20
<212>  DNA
<213>  Artificial Sequence

<220>
<223>  Actb primer reverse

<400>  4

atggctgggg tgttgaaggt                                                    20

<210>  5
<211>  25
<212>  DNA
<213>  Artificial Sequence

<220>
<223>  Actb primer forward

<400>  5
gacaacggct ccggcatgtg caaag                                               25

<210>  6
<211>  24
<212>  DNA
<213>  Artificial Sequence

<220>
<223>  Actb primer reverse

<400>  6
ttcacggttg gccttagggt tcag                                                24

<210>  7
<211>  20
<212>  DNA
<213>  Artificial Sequence

<220>
<223>  Prkcz primer forward

<400>  7
atgtctgctc ctccagcagt                                                     20

<210>  8
<211>  20
<212>  DNA
<213>  Artificial Sequence

<220>
<223>  Prkcz primer reverse

<400>  8
atatcctttc gctgcactgg                                                     20